# EUROPEAN PATENT SPECIFICATION

(11) **EP 0 934 073 B1**
(45) Date of publication and mention of the grant of the patent: **12.02.2003**
(21) Application number: 97929322.2
(22) Date of filing: 30.06.1997
(51) Int. Cl.: A61K 35/20, A61K 39/395, A61P 31/04

(54) **PHARMACEUTICAL COMPOSITION, COMPRISING COMPLEMENT PROTEINS, FOR THE TREATMENT OF HELICOBACTER INFECTIONS AND A METHOD FOR THE PREPARATION OF THE COMPOSITION**
PHARMAZEUTISCHE ZUSAMMENSETZUNG, ENTHALTEND KOMPLEMENTPROTEINE, ZUR BEHANDLUNG VON HELICOBACTER-INFEKTIONENSOWIE EIN VERFAHREN ZUR HERSTELLUNG DER ZUSAMMENSETZUNG
COMPOSITION PHARMACEUTIQUE, COMPRENANT DES PROTEINES DE COMPLEMENT, DESTINEE AU TRAITEMENT DES INFECTIONS A HELICOBACTER, ET PROCEDE DE PREPARATION DE LA COMPOSITION

(30) Priority: 28.06.1996 FI 962687; 28.06.1996 FI 962688
(43) Date of publication of application: 11.08.1999
(73) Proprietor: VALIO OY, 00370 Helsinki (FI)
(72) Inventor: KORHONEN, Hannu, FIN-11120 Riihimäki (FI); SYVÄOJA, Eeva-Liisa, FIN-02200 Espoo (FI); VASARA, Erkki, FIN-12240 Hikiä (FI); KOSUNEN, Timo, FIN-00930 Helsinki (FI); MARNILA, Pertti, FIN-20540 Turku (FI)
(74) Representative: Karvinen, Leena Maria
(86) International application number: FI9700418
(87) International publication number: WO98000150

(56) References cited:
- EP-A- 0 253 395
- EP-A- 0 338 229
- EP-A- 0 484 148
- EP-A- 0 556 083
- WO-A-95/08562
- File WPI, Derwent Accession No. 94-299805, SNOW BRAND MILK PROD. CO. LTD., "Prodn. of Pathogen Adhesion-Inhibiting Complement C3c, Useful in Food, Feed, Drugs-by Contacting C3c-Contg. Milk Material with Cation Exchanger, Eluating C3c from Exchanger and Opt. Adsorbing on Anionic Exchanger"; & JP,A,06 228 200, 16-08-94.
- DIALOG INFORMATION SERVICE, File 155, Medline, Dialog Accession No. 08317603, Medline Accession No. 95340389, KORHONEN H. et al., "Bactericidal Effect of Bovine Normal and Immune Serum, Colostrum and Milk Against Helicobacter Pylori"; & J. APPL. BACTERIOL., (ENGLAND), Jun. 1995, 78(6), p655-62.

## Description

The invention relates to a pharmaceutical composition to be used for the treatment of *Helicobacter* infections in mammals, including human, especially infections of gastric mucosa caused by *Helicobacter pylori*, and a process for the preparation of such a composition.

*Helicobacter pylori* is nowadays considered as the main cause of the genesis of gastric or duodenal ulcer and, in addition, more and more evidence has been obtained of the fact that gastritis induced by *H. pylori* may be an essential initial phase of the genesis of gastric cancer (Nomura *et al*. 1993).

One of the most striking characteristics of *H. pylori* is its exceptionally high urease activity. Urease is weakly attached to the cellular membrane of the bacterium and it forms over 5 % of the soluble proteins of the bacterium. Urea changes into ammonia by the action of urease. It has been thought that the ammonia produced by the urease enzyme protects helicobacter against the detrimental effect of gastric acids (Danon *et al*. 1995). The acid resistant urease enzyme on the outer membrane of helicobacter degrades the urea present in the stomach and in the gastric mucosa and protects by this way the bacterium by an ammonium ion cloud (Mobley *et al*. 1991).

Kₘ for urea of the *H. pylori* urease is 0.8 mM (Mobley *et al*. 1988), whereby it binds a substrate with much a greater affinity than the ureases of other bacterial families.
The urea concentration of the stomach has been found to be 1-14 mM (Kim *et al*. 1990). As the specific activity of the urease enzyme of *H. pylori* is very high, this bacterium is able to hydrolyze the fairly small amount of urea present in the stomach. People suffering from helicobacter infection have ammonia in their gastric juice on the average 0.015 %, or a concentration of about 10 mM (Tsujii *et al.* 1995). Urease has also been suggested to be an important virulence factor in the pathogenesis of *H. pylori* (Mobley *et al*. 1991).

It has been known for a long time that ammonia inhibits complement activity (Gordon *et al*. 1926). The thioester bond of the third component (C3) of the complement cascade is very susceptible to a nucleophilic attack by ammonia (Pangburn and Müller-Eberhard 1980). When ammonia reacts with the thioester bond, hydrolysis follows which results in a slow change of conformation, whereby a form of C3 is created which has all the other characteristics of C3b, except that it lacks the thioester binding site, whereby it only exists in the liquid phase. Thus C3 slowly gets inactivated by the action of ammonia. Also the C4 component of the complement contains a thioester bond, which is cut by the action of ammonia, whereby C4 loses its activity (Pangburn 1992, Dodds *et al*. 1996). By the normal plasma concentrations (12 µM) of ammonia this nucleophil causes an inactivation which is only 0.005 % / C3 / h, and the direct hydrolysis caused by water has much a greater influence than ammonia has, whereby a C3 molecule whose action is similar to that of C3b is created in the plasma (Pangburn & Müller-Eberhard 1980). According to the present understanding the continuous slow formation of C3(H₂O) causes the activation of the spontaneous alternative reaction pathway of the complement.

We suppose that in the gastric mucosa infected by helicobacter organisms the circumstances are different from those in the circulation. As the diffusion in the viscous matrix of the mucosa is slow, the local concentrations of the urease enzyme, and thus of its product, ammonia, can increase to be substantially high, especially in close vicinity to helicobacter colonies and also close to the cell membrane of individual helicobacter organisms. Pangburn and Müller-Eberhard published in 1980 a study according to which 50 mM of ammonia is able to cause 50% inactivation of C3. People suffering from helicobacter infection have ammonia in their gastric juice in the average about 10 mM (Tsujii *et al*. 1995), and thus the local concentrations in the gastric mucosa can very well be tenfold. As in the viscous matrix of the gastric mucosa the diffusion of the large C3 molecules is fairly slow, the amount of ammonia produced by the urease secreted by helicobacter is probably high enough to hydrolyze a sufficient amount of C3 to prevent the function of the classical reaction pathway of the complement so much that helicobacter survives well in the gastric mucosa. C3, whose thioester bond is hydrolyzed, does not harm the helicobacter, as the spontaneous activation of the alternative reaction pathway of the complement elsewhere than on the surface of the bacterium does not damage helicobacter.

Helicobacter tolerates well in *in vitro* circumstances the ammonia it produces. Not even 85 mM of ammonia did reduce the viability of *H. pylori* (Clyne *et al*. 1995). Concentrations higher than this were not tested by Clyne *et al*. It is thus probable that ammonia is not toxic to helicobacter itself and so does not prevent the bacterium to produce it to the glandular cavities of the stomach in so high concentrations that they would be sufficient to suppress the immune reactions mediated by the complement, which would result in local immune deficiency. This view is strongly supported by a recently published Norwegian study (Berstad *et al*. 1997), where tissue samples of the gastric mucosa of gastritis patients were studied using immunohisto-chemical methods and it was found that on the surface of helicobacter cells present in the glandular cavities of gastric mucosa there are no complement molecules, and the cells are intact, whereas on the surface of helicobacter cells which are "exposed", elsewhere at the entrances of the glandular crypts or outside them there are complement proteins, and also plenty of dead bacteria appeared in said areas. The concentration of ammonia can rise to be high especially in the glandular cavities of the gastric mucosa, and spreading of complement proteins into them is correspondingly slower.

The individuals having *H. pylori* infection have often a high titre of anti-*H. pylori*-antibodies but the immune system is not, however, capable of total destruction (eradication) of *H. pylori.* It has been found in numerous *in vitro* studies that the complement of blood and tissue fluids is alone able to kill *H. pylori* efficiently (Tosi and Czinn 1990, Pruul *et al*. 1987). *H. pylori* is not able to spread from the gastric mucosa into blood because then the complement kills it immediately. Leukocytes are able to phagocytosis of *H. pylori* if it has been opsonized only by antibodies, but they fail to kill it. If *H. pylori* has been opsonized by the components of the complement, mainly by C3b, *H. pylori* will die after phagocytosis (Das *et al*. 1988, Andersen *et al*. 1993, Kist *et al*. 1993). The complement is a more effective opsonine against helicobacter than antibodies (McKinley *et al*. 1993) and it is able to opsonize helicobacter well even without the presence of antibodies (Bernatowska *et al*. 1989).

Both the alternative reaction pathway of the complement, independent on antibodies, and the classical reaction pathway mediated by antibodies, kills helicobacter organisms effectively (Rautelin *et al*. 1993). Korhonen *et al*. 1995 suggested that the alternative reaction pathway of bovine complement would not kill helicobacter, but our later results show to the contrary. The reason to the earlier results is the fact that many commercially available fetal calf serum samples have been stored at too high a temperature (-20 °C) and thawed inbetween for sterile filtering, whereby the complement which is susceptible to destruction when thawed, or stored at higher than -70 °C, has lost its activity. Fresh fetal calf serum in which the complement has thus remained active, kills helicobacter.

As a summary of various *in vitro* studies it can be stated that any other of the natural defence mechanisms of the organism than the complement have not been shown to have alone an ability to kill *H. pylori* effectively. Instead of that, when complement factors are present, although in rather minor amounts, also other defence factors as e.g. phagocytes, are effective against *H. pylori* (Pruul *et al*. 1987, Kist *et al*. 1993, Rautelin *et al*. 1993).

Because complement seems to have a crucial role in the function of the natural defence mechanisms of the organism against *H. pylori,* a preparation, with which the natural immunity of the gastric mucosa could be strengthened, would be very useful in the treatment of helicobacter infections. We have studied bovine colostrum in order to develop such a product.

We have noticed already earlier (Korhonen *et al*. 1995) that bovine colostrum kills helicobacter very efficiently. Immunizing of a bovine animal is not a prerequisite for this function of killing helicobacter, and it is thus not dependent on helicobacter-antibodies present in milk. On the basis of our investigations we consider that the best result would be achieved with a preparation, to which plenty of complement proteins have been enriched in a process which has been designed specifically keeping an eye on the survival of the activity of the complement proteins. The preparation can also include antibodies but they are not essential, because the main factor killing the helicobacter is the complement included in the milk.

We have now been successful in developing from colostrum a product, so called "complement milk powder" into which plenty of active complement has been enriched.

The product according to the invention is preferably prepared from the colostrum of a cow by enriching the complement fraction of the colostral whey using the process we have developed, which is based on chromatography and membrane technology. Biologically active agents, e.g. lactoperoxidase and lactoferrin have even earlier been isolated from milk raw material using cation chromatography at low temperatures (e.g. EP-A-556083 and EP-A-253395). It is also known that whey proteins can be fractionated chromatographically with ion exchange resins. Our process is, however, different from the known chromatographic processes in its details. Because the complement included in the raw material has to be retained in active form, it is very important, at all of the process stages, from the storage of the raw material on, to keep the material to be handled at a temperature as low as possible to avoid inactivation of the complement. As a raw material in the enrichment process besides of colostrum also common milk or blood can be used.

In an advantageous process according to the invention the colostrum is first heated to the separation temperature (37 °C). The heated colostrum is separated or, alternatively, centrifuged to remove fat. If the colostrum has been stored as frozen, it is thawed before the separation in a vessel provided with a blender and a mantle.

The casein of the fat-free colostrum so obtained is coagulated by chymosin enzyme (Chy-Max, Pfizer Inc., Wisconsin, USA, or alike). It is important that pH remains approximately as neutral. Thus acid coagulation cannot be used because low pH destroys the complement. The mass is cut and the whey-casein mixture is transferred to a bag centrifuge to separate casein from whey. Alternatively, whey can be separated by a cheese press or a cheese cloth. If desired, the lactose of the whey is degraded by lactase enzyme to glucose and galactose. After adding lactase the whey is cooled under 10 °C. Alternatively, lactose hydrolysis can be omitted. The meaning of lactose degradation is to improve the separation of the complement proteins in the chromatography column.

An alternative, preferable way to pretreat the colostrum before chromatography is to add the lactase enzyme to the colostrum right away, when using frozen colostrum already when thawing it, and carry out the enzymatic coagulation of the casein right after this, e.g. at about 30 to 32 °C for 30 mins, and only after that carry out the separation of the whey. Then a portion of the fat is removed already with the casein, and when the whey obtained is separated, besides the residual fat also the casein dust can be removed from it. An advantage of this alternative procedure is e.g. the fact that one can act at temperatures as low as possible, and the above mentioned separation temperature of 37 °C is not needed at all.

The material pretreated as described above and cooled to about 10 °C is subjected to chromatography. The chromatographic method used is based on the separation of proteins from the other particles of whey on the basis of molecular size. Large molecules flow quickly through the column, whereas small molecules penetrate into the resin particles. So their flowing time in the column increases. On the basis of this property of the resin the macromolecular proteins, e.g. the complement, which are typical to the colostrum, can be clearly separated to a fraction of their own from lactose and its monosaccharides.

The chromatography column is filled with an ion exchange resin which is preferably a strong cation exchange resin in Na⁺ form, and is equilibrated with whey (the amount of whey at least 10 times the volume of the resin). To sterilize the column and to prevent the bacterial growth the column is washed with 80 °C water for about 2 h. After this the column can be cooled with 4 °C water, and finally the surface of the water in the column is lowered to the upper level of the resin.

After this the whey batch can be fed into the column. Water is used as the eluent in the column with a fow rate of about 20 l/h. The pH of the resin is 7. The elution of the proteins and sugars can be monitored e.g. with a Brix gauge (Atago Co. Ltd., Japan), with conductibility measuring (e.g. Ciba Coming Analytical, Mettler Toledo Ltd., UK) and with the Clini tablets or strips (Clinitest, Bayer Diagnostics, UK) intended for measuring of glucose. Alternatively, the elution can be monitored spectrophotometrically in the wavelength of 280 nm.

The protein solution diluted in the column can be concentrated by reverse osmose (cut-off of the membrane 500-5000) and so lessen the need of freeze-drying capacity. The reverse osmose membrane is so tight that only water molecules pass through it. Using reverse osmose the dry matter content of the solution can be increased up to 10 to 30 %. Typically the concentration factor is 2-3, i.e. concentration of 1:2 to 1:3 with regard to the liquid volume is carried out. The filtering pressure used is 10 to 40 bar.

The concentrated solution is sterilized by filtering. Then commercially available 0.22 µm or 0.45 µm membrane filters can be used, or e.g. the Bactocatch filtering equipment by Alfa-Laval. The purpose of sterile filtering is to replace pasteurization in the enrichment process. Pasteurization or other heat treatments cannot be used to destroy the bacteria, because high temperatures would also lead to denaturation of the complement.

Typically the sterile filtering is carried out through 0.22 µm or 0.45 µm membranes. The membrane holds the bacteria but other whey components pass through it.

The concentrated, sterile protein solution is administered on freeze dryer plates which arc frozen and packed in the freeze dryer. The freeze drying process takes typically for 2-3 days. The freeze dried protein powder is packed e.g. into aluminum bags using an automated packing machine. The size of a finished package is typically 5-10 g. The powder keeps long times as frozen at -70 °C but it can be stored also at a normal freezer temperature, at about -20 °C, and for some time even at a refrigerator temperature, about +4 °C.

The bactericidal activity of the complement fraction enriched according to the process of the invention as 20% (w/v) solution in a buffer solution is higher than the activity of the colostral whey used as the starting material, and about the same as the activity of serum. As the final product the complement fraction is a white, hygroscopic, but easily water-soluble powder. The taste of the powder is mildly sweet. On the basis of these properties the powder is well suitable to be mixed with different semifinished products of food industry, sweets and pharmaceutical preparations.

To find out if the *in vivo* activity of the complement preparation is exclusively dependent on the activity of the complement proteins, or if the activity is also dependent on the specific antibodies contaminating the product, we studied the activity of the colostral whey powders prepared according to the process as described above from the colostrum of cows immunized with *Helicobacter felis*, and non-immunized cows, respectively, both in a bactericidal test in *in vitro* circumstances and in the treatment of the mouse experimental helicobacter infection (*in vivo*). The treatment of two weeks with both colostral whey powder of non-immunized cows (T-powder = the complement milk powder of the invention) and colostral whey powder of immunized cows (IT-powder) decreased the colonization of helicobacter in gastric mucosa (Example 3). As the powder which is made of colostral whey of an immunized cow and contains a great concentration of specific antibodies was not more active than the product which does not contain specific antibodies, the bactericidal activity of the complement included in the preparations was an essential characteristic in view of the treatment activity.

The complement milk powder containing active bovine complement both kills helicobacter as such (the effect of the complement) and restores the local immune deficiency caused by helicobacter by supporting the own humoral and cell mediated immune response of the gastric mucosa, not only against helicobacter but also against other pathogens. So the bacterial flora of the stomach normalizes and intestinal pathogens disappear when the state of immune deficiency in the stomach is restored. When the growth of helicobacter is prevented, the production of ammonia in the gastric mucosa is also running out, and so the disadvantages caused by ammonia, as e.g. carcinogenesis, are prevented.

In order to increase the activity of the complement, and to improve the treatment capacity, serum, or a powder obtained from blood serum by freeze drying, or a complement concentrate obtained from blood serum by fractionating, can also be added into the complement milk powder of the invention. To improve the treatment capacity, antibiotics to which the helicobacter organisms to be controlled are sensitive, can also be added into the complement milk powder of the invention.

### Brief description of the drawings

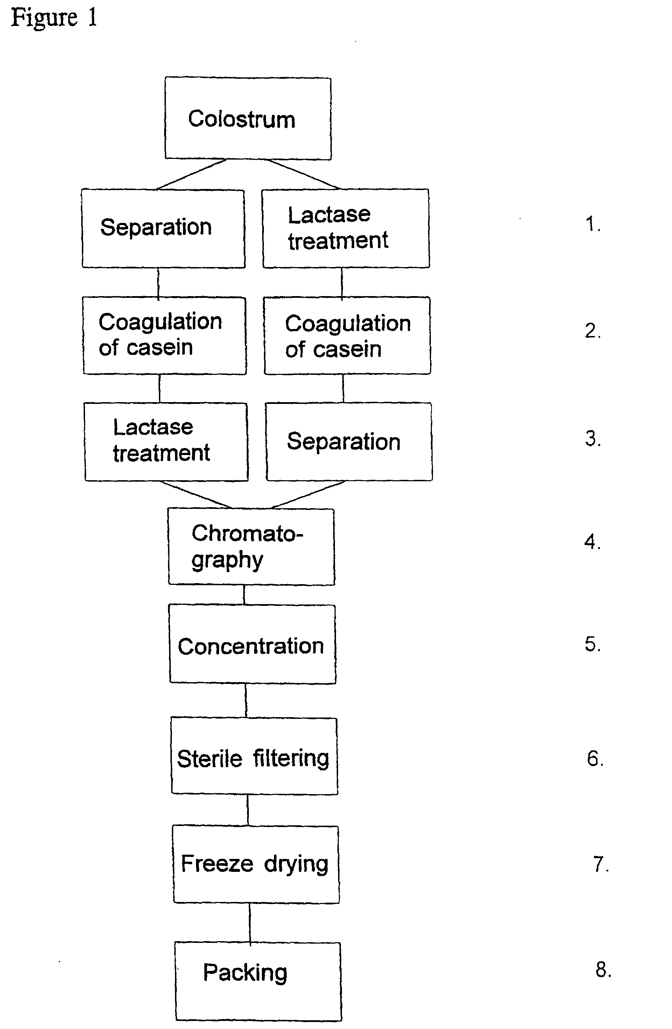

- **Figure 1**: The process chart of the enrichment process of the invention wherein the alternative pretreatment phases are described.

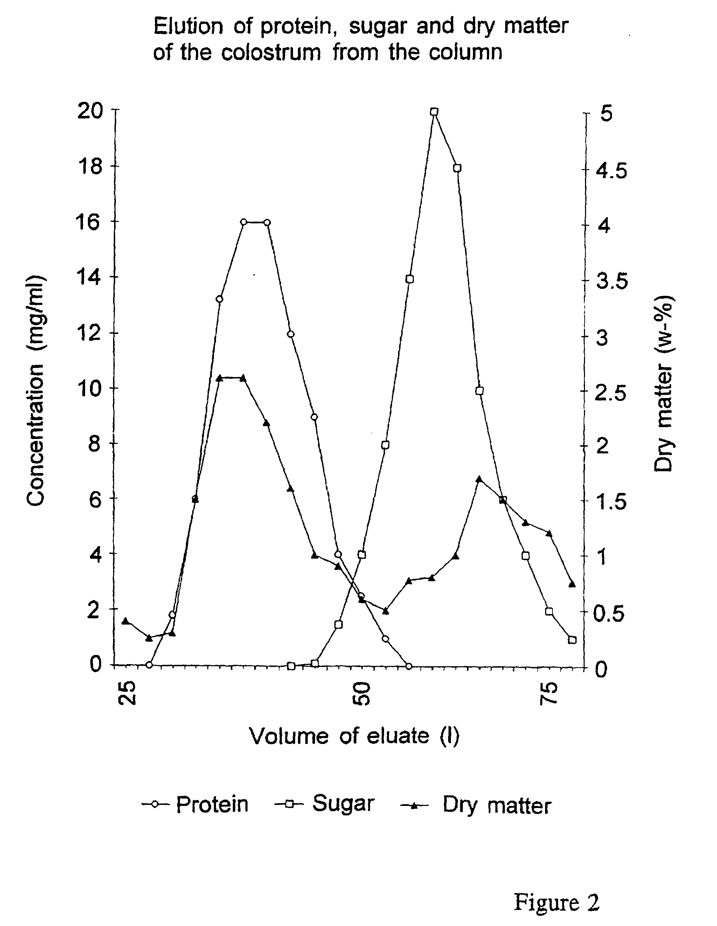

- **Figure 2**: Elution of the proteins, sugars and the dry matter of the colostrum in the chromatographic separation carried out according to the invention. The sugar was measured as glucose by Clinitest.

In the following examples the performance of the enrichment process of the invention is described in detail, as well as the bactericidal activity of the product of the invention, i.e. the complement milk powder, obtained by the process. Colostral whey used as the starting material and colostral whey powder obtained from immunized cows were used as reference materials.

### Example 1 Performance of the enrichment process of the invention

### a. Pretreatment of colostrum

### ALTERNATIVE A

Deep-frozen colostrum of non-immunized or immunized cows was thawed in a vessel provided with a blender and a mantle and was heated to the separation temperature (37 °C). The heated colostrum was separated to remove fat (Alfa-Laval LAPX 202 separator). Casein of the fat-free colostrum was coagulated by chymosin enzyme (Chy-Max, 1 ml/1, 37 °C/30 min), the mass was cut and the whey-casein mixture was transferred to a bag centrifuge (Carl Padberg 1971) to separate casein from the whey. The lactose of the whey was degraded by lactase enzyme (Maxi-Lact 900 µl/l) to glucose and galactose. After adding the lactase the whey was cooled to be under 10 °C.

### ALTERNATIVE B

Deep-frozen colostrum was thawed in a vessel provided with a blender and a mantle, and lactase enzyme (Maxi-Lact 900 µg/l) was added thereto. The colostrum was heated to the temperature of 30-32 °C, chymosin enzyme (Chy-Max, 1 ml/l) was added thereto, and it was kept at the above indicated temperature for 30 mins. The coagulated casein mass was cut and the whey-casein mixture was transferred to a bag centrifuge (Carl Padberg 1971) to separate casein from the whey. The whey so obtained was separated (Alfa-Laval LAPX 202 separator) to remove the residual fat and casein dust, without heating the whey any longer. The separated whey was cooled to be under 10 °C.

### b. Chromatographic separation

The height of the column was 2000 mm and the diameter 256 mm. The column was filled (about 100 l) with strong cation exchange resin in Na⁺ form (Finex VO 7 C resin, Cultor Oy) and equilibrated with whey (the amount of whey 10 times the resin volume). To sterilize the column and to prevent bacterial growth the column was washed with 80 °C water for 2 h. After this the column was cooled and equilibrated with 4 °C water, and finally the surface of the water in the column was lowered on the upper level of the resin.

After this a whey batch of 10 litres was fed into the column. Water was used as the eluent in the column, flow rate 20 l/h.

The elution of the proteins and sugars was monitored with conductibility measuring (Ciba Corning Analytical, Mettler Toledo Ltd., UK) and with the Clini tablets or strips (Clinitest, Bayer Diagnostics, UK) intended for measuring of glucose.

### c. Concentration with reverse osmose

The protein solution diluted in the column was concentrated by reverse osmose (cut-off of the membrane 5000, type AFC 40, Person Candy International, UK). The reverse osmose membrane was so tight that only water molecules passed through it. The dry matter of the solution was increased up to 20 %. The concentration factor was 3.

### d. Sterile filtering

The concentrated protein solution so obtained was sterilized by filtering it through 0.45 µm membrane (Pellicon HVMP 000 C5, Millipore Ltd., USA).

### e. Freeze drying

The concentrated, sterile protein solution was administered on sterilized freeze dryer plates which were frozen and packed in the freeze dryer (Usifroid SM.H 1515). The freeze drying process took 2 days.

### f. Packing and storing

The freeze dried powder was packed using an automated packing machine into aluminum bags. The size of a finished package was 6 g. As a protective gas 5% nitrogen was used. The packages were stored at -70 °C.

### Example 2

The significance of the complement, the immunization of cows, and the titres of the antibodies to the bactericidal activity of colostral whey, colostral whey powders and serum against *H. pylori.*

### Materials and methods

### Immunization of cows

*Helicobacter pylori* strain NCTC 11637 was used for immunization. The bacteria were cultivated on horse blood agar plates at 37 °C in microaerophilic conditions for 2 days as described in Höök *et al*. (1989). The bacteria were killed using 0.5% formalin, washed and suspended in sterile saline to a concentration of 2 x 10⁹ / ml. The vaccine included 10 % (v/v) of bacterial suspension and 90 % (v/v) of aluminum hydroxide adjuvant. Pregnant Friesian dairy cows were immunized with 4 ml of the vaccine intramuscularly in both sides of the neck. The immunization was started in the beginning of the dry period of the cows about two months before the calculated calving. The cows received 4 booster injections (2 ml in both sides of the neck) with one week intervals beginning after two weeks from the first immunization. The cows were healthy and they were not given antibiotics.

### Colostrum samples (colostral whey)

Casein of the colostrum samples was coagulated with chymosine (1h at 37 °C), after which the casein fraction was removed. Lactose was degraded with lactase enzyme and fat was separated by centrifugation and discarded, whereby the colostral whey which was used in the comparative tests was retained.

### Serum samples

Venous blood samples were obtained from two cows before immunization with *H. pylori* strain NCTC 11637 as described above, and after immunization. The blood samples were let to coagulate at room temperature for about one hour. The serum was separated from the coagulated blood by centrifugation (4000 rpm, 20 min), whereafter the serum was deep-frozen to -70 °C. So control sera (KS) and immune sera (IS) were obtained from the same animals.

### Determination of specific antibodies

The amounts of the antibodies specific to helicobacter of the colostral whey, the colostral whey powders and the sera of the cows were measured by ELISA method, as described in Rautelin and Kosunen (1987). In the method wells of a microtitre plate were coated with an antigen extracted from *Helicobacter pylori* (the antigen was dissolved in phosphate buffered saline in the concentration of 1 µg/ml) overnight. The wells were washed, whereafter different dilutions made from the samples were incubated in the wells overnight. After washing the specific antibodies remained in the wells were determined by adding goat-anti-bovine-IgG-antibody having alkaline phosphatase label. The amount of the conjugate fastened in the well was measured fotometrically. The amount of helicobacter-antibodies of the samples were detected as a cut-off value of the absorbance 0.5 OD₄₀₅.

### Measuring the bactericidal activity

The bactericidal activity was measured with the so called plate counting method. *H. pylori* strain NCTC 11637 was cultivated in horse blood agar plates in microaerophilic conditions for 2 days as described in Höök *et al*. (1989). The bacteria were rinsed from the plate with sterile Tris-buffered (0.05M) saline (pH 7.2), washed once with (3000 rpm, 7 min) and suspended in Tris-buffer. The number of the bacteria was adjusted to about 10⁵ to 10⁶/ml. Fetal calf serum was diluted to 10% (v/v) in the above Tris buffer. The colostral whey samples were diluted to 50% (v/v) and the complement milk powders were dissolved to 20% (w/v) in Tris buffer. The serum samples were diluted to 20% (v/v) in Tris buffer. The above mentioned solutions were sterilized before the test by 0.45 µm filter. The inactivation of the complement of the colostral whey, colostral whey powders and serum was carried out by heating the sample to 56 °C for 30 minutes. As the source of complement in the heated samples fetal calf serum which contains complement but no antibodies was used. The colostral whey samples and the colostral whey powders were tested for possible antibiotic residues by the Delvotest method. All samples turned out to be negative.

The bactericidal test was carried out so that 0.5 ml of a sample, 0.25 ml of 10% fetal calf serum (or Tris buffer) and 0.25 ml of bacterial suspension were mixed in a sterile test tube. A 0.1 ml sample was taken from the tube for the 0-hour bacterial counting. The rest of the reaction mixture was incubated for 2 hours at 37 °C in microaerophilic conditions. When the incubation was completed, a 0.1 ml sample of the reaction mixture was taken, from which a dilution series was made immediately into sterile Tris buffer. The colony forming units of *H. pylori* were determined from the dilutions on horse blood agar using the so called drop techniques. The colonies on the plates were counted after 2-3 days cultivation.

Each test included the following combinations:
- Sample + buffer
- Buffer + *H. pylori*
- Sample + buffer + *H. pylori*
- Complement-inactivated sample + *H. pylori*
- Complement-inactivated sample + fetal calf serum + *H. pylori*
- Fetal calf serum + *H. pylori*

### Results

**Table 1a.**

| Bactericidal activity against *H. pylori* of the whey samples prepared of the first milking of non-immunized cows. | | | |
|---|---|---|---|
| Cow | Colostral whey | Complement-inactivated colostral whey*) | Complement-inactivated colostral whey*) + fetal calf serum |
| 1 | +++ | -- | +++ |
| 2 | +++ | -- | ++ |
| 3 | +++ | -- | ++ |
| 4 | +++ | ++ | +++ |
| 5 | +++ | ++ | +++ |
| -- = no change, + = >10¹, ++ = >10³ and +++ = >10⁵ decrease in the number of growing bacteria / ml. | | | |

| | | | |
|---|---|---|---|
| *) heated at 56°C for 30 min. | | | |

**Table 1b.**

| Bactericidal activity against *H. pylori* of the whey samples prepared of the first milking of cows immunized with *H. pylori.* | | | |
|---|---|---|---|
| Cow | Colostral whey | Complement-inactivated colostral whey*) | Complement-inactivated colostral whey*) + fetal calf serum |
| 1 | + | -- | ++ |
| 2 | ++ | -- | ++ |
| 3 | -- | -- | ++ |
| 4 | -- | -- | ++ |
| 5 | + | -- | ++ |
| 6 | -- | -- | ++ |
| -- = no change, + = >10¹, ++ = >10³ and +++ = >10⁵ decrease in the number of growing bacteria / ml. | | | |

| | | | |
|---|---|---|---|
| *) heated at 56°C for 30 min. | | | |

**Table 2.**

| Titres of the specific *H. pylori* antibodies in the samples prepared from the colostrum of the first milking of immunized and non-immunized cows. | |
|---|---|
| Immunized cow | Non-immunized cow |
| 1 6,000 | 1 <100 |
| 2 5,800 | 2 <100 |
| 3 14,000 | 3 <100 |
| 4 4,600 | 4 <100 |
| 5 3,500 | 5 <100 |
| 6 27,000 | - |

**Table 3.**

| Bactericidal activity of IT- and T-powders against *H. pylori*. The concentration of the whey powder in the test was 10 % (w/v). The titre of the specific helicobacter-antibodies in the IT powder was 10,000. | | | | |
|---|---|---|---|---|
| Powder | Colostral whey powder | Complement-inactivated colostral whey powder*) | Complement-inactivated colostral whey powder*) + fetal calf serum | Fetal calf serum |
| T-powder | +++ | -- | ++ | ++ |
| IT-powder | +++ | -- | ++ | ++ |
| -- = no change, + = >10¹, ++ = >10³ and +++ = >10⁵ decrease in the number of growing bacteria / ml | | | | |

| | | | | |
|---|---|---|---|---|
| *) heated at 56 °C for 30 min. | | | | |

**Table 4.**

| Bactericidal activities against *H. pylori* of control sera (KS) obtained from two cows before immunization with *H. pylori,* and immune serum samples (IS) obtained from the two cows after immunizations. Titres of specific helicobacter-antibodies of said serum samples are given in parenthesis. | | | |
|---|---|---|---|
| Cow | Serum | Complement-inactivated serum*) | Complement-inactivated serum*) + fetal calf serum |
| 1 KS (<300) | ++ | -- | +++ |
| 1 IS (24,000) | ++ | -- | +++ |
| 2 KS (<300) | + | -- | + |
| 2 IS (30,000) | + | -- | + |
| -- = no change, + = >10¹, ++ = >10³ and +++ = >10⁵ decrease in the number of growing bacteria / ml | | | |

| | | | |
|---|---|---|---|
| *) heated at 56 °C for 30 min | | | |

It appears from the results given in Tables 1a and 1b that in eight cows of eleven the colostrum contains the active complement which kills helicobacter. In six of these the bactericidal activity of the colostral whey is exclusively dependent on the complement, because heating which inactivates the complement destroys also the bactericidal activity, and adding of complement using fetal calf serum restores the bactericidal activity. In the concentration used in this test fetal calf serum was not as such bactericidal against *H. pylori* because fetal serum lacks antibodies, and the classical reaction pathway of complement which is independent on antibodies requires rather high complement concentration to be bactericidal (serum concentration over 5 % v/v). The complement of the commercial fetal serum sample used is mostly inactivated because the serum has been stored at -20 °C. However, when added into colostral whey samples which had not inherently a sufficient bactericidal activity against *H. pylori*, fetal calf serum always caused a strong bactericidal effect. The reason to this was the fact that colostrum, also that of non-immunized cows, contains somewhat of the necessary antibodies to activate the classical reaction pathway of the complement. Complement proteins are able to kill bacteria in smaller concentrations (serum concentration under 5 % v/v) using the classical reaction pathway than by the alternative reaction pathway.

The bactericidal activity of colostrum does not depend on the immunization of cows against hclicobacter, or the titre of specific antibodies (Table 2).

The results given in Table 3 show that in the "complement milk powder" (T-powder) of the invention, and in the "immune whey powder" (IT-powder) obtained from the colostrum of immunized cows the complement proteins enrich and retain well their bactericidal activity against helicobacter. The concentration of specific antibodies is not substantial in view of the bactericidal activity. In this test also fetal calf serum alone killed helicobacter organisms, because the sample used was fresh and stored at -70 °C.

The results given in Table 4 show that also serum complement has bactericidal activity against helicobacter. Bactericidal activity is neither in this case dependent on the immunization of the cows against helicobacter, or the titre of the specific antibodies. Serum or complement proteins enriched and freeze dried therefrom can in the light of these results also be used as complement source in the treatment of helicobacter infection either as one component of the colostral whey powder, or alone.

### Example 3

*In vivo* test concerning the activity of the colostral whey powders of non-immunized cows and of cows immunized with a *Helicobacter felis* strain in the treatment of mice infected with *H. felis* CS¹.

### Materials and methods

### Immunizations, and colostral whey powders

The *Helicobacter felis* CS¹ bacteria used for immunization were cultivated on horse blood agar plates at 37 °C in microaerophilic conditions for 2 days as described in Höök *et al*. (1989). The bacteria were killed with 0.5% formalin, washed and suspended in sterile saline to a concentration of 2 x 10⁹/ml. The vaccine contained 10 % (v/v) of bacterial suspension and 90 % (v/v) aluminum hydroxide adjuvant. Two pregnant Friesian dairy cows were immunized with 4 ml of the vaccine intramuscularly in both sides of the neck. Immunization was started in the beginning of the dry period of the cows about two months before the calculated calving. The cows received 4 booster injections (2 ml in both sides of the neck) with one week intervals beginning after two weeks from the first immunization. The cows were healthy and they were not given antibiotics during the immunization.

According to the procedure of Example 1 (Alternative A) colostral whey powder was prepared from the colostrum of the first three milkings, both from the colostrum of a non-immunized cow (T-powder = complement milk powder of the invention) and from the colostrum of two cows immunized with *H. felis* CS¹ bacteria (IT-powder). The amounts of the specific helicobacter-antibodies were determined as described in Example 2. The titre of the IT-powder was 2900 and that of the T-powder was below 10.

### H. felis bacteria used for infecting mice

The strain *Helicobacter felis* CS¹ was grown on horse blood agar plates at 37 °C in microaerophilic conditions for 2 days as described in Höök *et al*. (1989). The bacteria were rinsed from the plate with sterile phosphate buffered saline (pH 7.0), washed once with (3000 rpm, 7 min) and suspended in phosphate buffer. The number of the bacteria was adjusted to about 10¹⁰/ ml.

### Test arrangement

30 female Balb C mice (age 6-7 weeks) were infected with *H. felis* CS¹ bacteria by giving them orally in three successive days 10⁹ *H. felis* CS¹ bacteria in 0.1 ml of phosphate buffered saline. After one week from the last injection the treatment with the above mentioned colostral whey powders T and IT was begun. Ten mice were not treated at all, and they were infected controls. The treatment continued for two weeks. During the treatment the mice received orally two times a day 0.3 ml of Tor IT-powder dissolved in water (1 g of the lyophilisate in 3 ml of water, 29 % (w/v)). Both T-powder and IT-powder were used to treat groups of ten mice. After the treatments there was a 4 week's time to wait for the growth of the *H. felis* bacteria which had survived the treatment. 4 weeks after finishing the treatment the animals were decapitated for histological studies.

### Histological studies

The stomachs of the mice were removed and prepared to longitudinal sections which were rinsed in sterile saline and stored in 10% formalin solution. From the antrum and corpus parts of the samples stained with giemsa and hematoxylin-eosin dyes *H. felis* colonization was determined in the scale of 0-3, wherein 0 represents a healthy sample.

### Statistics

The statistical significance of the results showing the level of *H. felis* colonization was tested by non-parametric Kruskall-Wallis one-way variance analysis, as the materials are not normally distributed and the results are given in a classifying scale.

### Results

**Table 5a.**

| Level of *H. felis* colonization in the gastric antra of mice after the treatment. Scale 0-3. Statistical significance gives the risk level (P) compared to infected controls. | | | |
|---|---|---|---|
| | Infected controls | T-powder treatment group | IT-powder treatment group |
| Mean | 2.00 | 1.25 | 1.28 |
| ± S.D. | 0.46 | 0.79 | 0.97 |
| Median | 2.00 | 1.25 | 1.00 |
| n | 8 | 10 | 10 |
| Significance (P) | -- | 0.041 | 0.061 |

**Table 5b.**

| Level of *H. felis* colonization in corpora of mice after the treatment. Scale 0-3. Statistical significance gives the risk level (P) compared to infected controls. | | | |
|---|---|---|---|
| | Infected controls | T-powder treatment group | IT-powder treatment group |
| Mean | 0.500 | 0.600 | 0.300 |
| ± S.D. | 0.612 | 0.699 | 0.675 |
| Median | 0.00 | 0.50 | 0.00 |
| n | 9 | 10 | 10 |
| Significance (P) | -- | not significant | not significant |

The corpora of the mucosa of the stomachs of the mice were poorly infected. Even in the infected controls the level of *H. felis* colonization was very low, and thus conclusions of the treatment capacity cannot be drawn from the results of Table 5b. Instead, the antra were infected pretty well and the treatment capacity of the IT- and T-powders can be seen clearly (Table 5a). The results show clearly that both the IT- and the T-powders decreased the colonization of *H. felis* in the antra of the stomachs statistically significantly. The result can be considered as an evidence of the bactericidal activity of the complement included in the powders against helicobacter also *in vivo* conditions. Most probably the activity would have been even more clear if the mice had been decapitated immediately after the treatment, without giving the helicobacter cells four weeks time to regrow in the mucosa.

The amount of the specific antibodies (i.e. immunization of the cows) had no effect in this case either. Instead, oral administration of active bovine complement directly to the stomach is in the light of the results sufficient to remedy the immune deficiency caused by ammonia produced by the urease of helicobacter organisms so much that a clear treatment capacity can be seen.

### References

Andersen, L. P., Blom, J. and Nielsen, H. (1993); Survival and ultrastructural changes of *Helicobacter pylori* after phagocytosis by human polymorphonuclear leukocytes and monocytes. APMIS 101, 61-72.

Bernatowska, E., Jose, P., Davies, H., Stephenson, M. and Webster, D. (1989); Interaction of *Campylobacter* species with antibody, complement and phagocytes. Gut, 30, (7), 906-911.

Berstad, A. E., Brandtzaeg, P., Stave, R. and Halstensen, T. S. (1997); Epithelium related deposition of activated complement in *Helicobacter pylori* associated gastritis. Gut, 40, 196-203.

Clyne, M., Labigne, A. and Drumm, B. 1995; *Helicobacter pylori* requires an acidic environment to survive in the presence of urea. Infect Immun. 63, 1669-1673.

Danon, S. J., O'Rourke, J. L., Moss, N. D., and Lee, A. (1995); The importance of local acid production in the distribution of *Helicobacter felis in* the mouse stomach. Gastroenterology, 108, 1386-1395.

Das, S. S., Karim, Q. N. and Easmon, C. S. F. (1988); Opsonophagocytosis of *Campylobacter pylori*. J. Med. Microbiol. 27, 125-130.

Dodds, A.W., Ren, X. D., Willis, A. C. and Law, S. K. (1996); The reaction mechanism of the internal thioester in the human complement component C4. Nature, 379, (6561) 177-179.

Gordon, J., Whitehead, H. R., and Wormall, A. (1926); The action of ammonia on complement: the fourth component. Biochem. J. 20, 1028-1035.

Höök, J., Blomberg, B., Danielsson, D. and Kosunen, T. (1989); Detection of antibody responses in rabbits hyperimmunized with *Campylobacter pylori*. APMIS, 97, 56-60.

Kim, H., Park, C., Yang, W., Lee, K., Kwon, S., Robey-Cafferty, S., Ro, J., and Lee, Y. (1990); The gastric juice urea and ammonia levels in patients with *Campylobacter pylori.* Am. J. Clin. Pathol. 94, 187-191.

Kist, M., Spiegelhalder, C., Moriki, T. and Schaefer, H.-E. (1993); Interaction of *Helicobacter pylori* (strain 151) and *Campylobacter coli* with human peripheral polymorphonuclear granulocytes. Zbl. Bact. 280, 58-72.

Korhonen, H., Syväoja, E.-L., Ahola-Luttila, H., Sivelä, S., Kopola, S., Husu, J. and Kosunen, T. U. (1995); Bactericidal effect of bovine normal and immune serum, colostrum and milk against *Helicobacter pylori.* J. Appl. Bacteriol. 78, 655-662.

McKinley, A. W., Young, A., Russell, R. I. and Gemmell, C. G. (1993); Opsonic requirements of *Helicobacter pylori.* J. Med. Microbiol. 38, (3), 209-315.

Mobley, H. L. T., Cortesia, M. J., Rosenthal, L. E., and Jones, D. (1988); Characterisation of urease from *Campylobacter pylori. J.* Clin. Microbiol. 26, 831 -836.

Mobley, H. L. T., Hu, L.-T. and Foxall, P. A. (1991); *Helicobacter pylori urease:* properties and role in pathogenesis. Scand. J. Gastroenterol. Suppl. 187, 39-46.

Nomura, A., and Stemmermann, G. N., (1993); *Helicobacter pylori* and gastric cancer. J. Gastroenterol. Hepatol. 8, 294-303.

Pangburn, M. K. and Müller-Eberhard, H. J. (1980); Relation of a putative thioester bond in C3 to activation of the alternative pathway and the binding of C3b to biological targets of complement. J. Exp. Med. 152, 1102-1114.

Pangburn, M. K. (1992); Spontaneous thioester bond formation in alpha 2-macro-globulin, C3 and C4. FEBS-Lett. 308, (3), 280-282.

Pruul, H., Lee, P. C., Goodwin, C. S. and McDonald, P. J. (1987); Interaction of *Campylobacter pyloridis* with human immune defence mechanisms. J. Med. Microbiol. 2, 233-238.

Rautelin, H. and Kosunen, T. (1987); *Campylobacter* etiology in human gastroenteritis demonstrated by antibodies to acid extract antigen. J. Clin. Microbiol., 26, 1944-1951.

Rautelin, H., Blomberg, B., Fredlund, H., Järnerot, G. and Danielsson, D. (1993).; Incidence of *Helicobacter pylori* strains activating neutrophils in patients with peptic ulcer disease. Gut, 34, 599-603.

Tosi, F. and Czinn, S. J. (1990); Opsonic activity of specific human IgG against *Helicobacter pylori.* J. Infect. Dis. 162, 156- 162.

Tsujii, M., Kawano, S., Tsuji, S., Takei, Y., Tamura, K., Fusamoto, H. and Kamada, T. (1995); Mechanism for ammonia induced gastric carcinogenesis in rats. Carcinogenesis, 16, (3), 563-566.

## Claims

1. A pharmaceutical composition having *in vivo* bactericidal activity against bacteria of the genus *Helicobacter*, which composition comprises, as a freeze-dried concentrate, enriched complement proteins isolated from milk, colostrum or blood, the complement proteins having retained their biological activity during isolation and enrichment, and being able to keep said activity during storage.

2. The pharmaceutical composition according to claim 1, wherein the complement proteins have been enriched from bovine colostrum.

3. The pharmaceutical composition according to claim 1, wherein the complement proteins have been enriched from vertebrate serum, or wherein the serum, or complement proteins enriched therefrom have been added into the composition according to claim 2.

4. The pharmaceutical composition according to the claims 1, 2 or 3, wherein the bacteria of the genus *Helicobacter* belong to the species *Helicobacter pylori, Helicobacter felis* or *Helicobacter heilmannii.*

5. The pharmaceutical composition according to any one of the claims 1 to 4, further comprising antibiotics.

6. Use of a pharmaceutical composition comprising, as a freeze-dried concentrate, enriched complement proteins isolated from milk, colostrum or blood, the complement proteins having retained their biological activity during isolation and enrichment, and being able to keep said activity during storage, for the manufacture of a medicament for oral treatment of *Helicobacter* infections in gastric mucosa in human or another mammal.

7. Use according to claim 6, wherein the bacteria of the genus *Helicobacter* belong to the species *Helicobacter pylori, Helicobacter felis* or *Helicobacter heilmannii.*

8. Use of a pharmaceutical composition comprising, as a freeze-dried concentrate, enriched complement proteins isolated from milk, colostrum or blood, the complement proteins having retained their biological activity during isolation and enrichment, and being able to keep said activity during storage, and further comprising antibiotics, for the manufacture of a medicament for oral treatment of *Helicobacter* infections in gastric mucosa in human or another mammal.

9. A process for enrichment of complement proteins from milk or colostral whey, or blood serum in biologically active form, comprising
- pretreating milk, colostrum or blood to collect the whey/serum, whereby colostral whey is obtained by (a) separating the fat from the colostrum, coagulating the casein of the fat-free colostrum with enzymatic coagulation, and degrading enzymatically the lactose included in the whey so obtained, or (b) by adding lactase enzyme to the colostrum, coagulating the casein with enzymatic coagulation, and separating the whey so obtained,
- feeding a batch of the whey/serum into a chromatography column filled with a cation exchange resin,
- eluting the protein fraction from the column with water under the temperature of 10°C, and
- sterilizing, concentrating and freeze drying the collected protein fraction to dry matter content of about 10 to about 30%.

10. The process according to claim 9, wherein the resin is strong cation exchange resin in Na⁺ form.

11. The process according to claim 9, wherein the resin is equilibrated with whey before feeding the whey batch into the column.

12. The process according to claim 9, comprising carrying out the concentration step with reverse osmose from 1:2 to 1:3 as regards the liquid volume.

13. The process according to claim 9, comprising storing the freeze-dried powder at -70 °C.

## Patentansprüche

1. Arzneimittel, das in vivo Bakterizidaktivität gegen Bakterien der Gattung *Helicobacter* hat, wobei das Mittel als gefriergetrocknetes Konzentrat angereicherte Komplementproteine umfasst, die aus Milch, Kolostrum oder Blut isoliert wurden und ihre biologische Aktivität während des Isolierens und Anreicherns beibehalten haben und während der Lagerung diese Aktivität aufrecht erhalten können.

2. Arzneimittel nach Anspruch 1, wobei die Komplementproteine aus Rinderkolostrum angereichert worden sind.

3. Arzneimittel nach Anspruch 1, wobei die Komplementproteine aus Wirbeltierserum angereichert worden sind oder wobei das Serum oder damit angereicherte Komplementproteine dem Mittel nach Anspruch 2 zugesetzt worden sind.

4. Arzneimittel nach Anspruch 1, 2 oder 3, wobei die Bakterien der Gattung *Helicobacter* der Art *Helicobacter pylori, Helicobacter felis* oder *Helicobacter heilmanii* angehören.

5. Arzneimittel nach einem der Ansprüche 1 bis 4, das ferner Antibiotika umfasst.

6. Verwendung eines Arzneimittels, das als ein gefriergetrocknetes Konzentrat angereicherte Komplementproteine umfasst, die aus Milch, Kolostrum oder Blut isoliert wurden und ihre biologische Aktivität während des Isolierens und Anreicherns beibehalten haben und während der Lagerung diese Aktivität aufrecht erhalten können, für die Herstellung eines Medikaments zur oralen Behandlung von *Helicobacter*-Infektionen der Magenschleimhaut des Menschen oder anderer Säugetiere.

7. Verwendung nach Anspruch 6, wobei die Bakterien der Gattung *Helicobacter* der Art *Helicobacter pylori, Helicobacter felis* oder *Helicobacter heilmanii* angehören.

8. Verwendung eines Arzneimittels, das als ein gefriergetrocknetes Konzentrat angereicherte Komplementproteine umfasst, die aus Milch, Kolostrum oder Blut isoliert wurden und ihre biologische Aktivität während des Isolierens und Anreicherns beibehalten haben und während der Lagerung diese Aktivität aufrecht erhalten können, und das ferner Antibiotika umfasst, für die Herstellung eines Medikaments zur oralen Behandlung von *Helicobacter*-Infektionen der Magenschleimhaut des Menschen oder anderer Säugetiere.

9. Verfahren zur Anreicherung von Komplementproteinen aus Milch oder Kolostrummolke oder Blutserum in biologisch aktiver Form, umfassend
- Vorbehandeln der Milch, des Kolostrums oder des Bluts, um die Molke/das Serum zu sammeln, wobei die Kolostrummolke erhalten wird durch (a) Trennen des Fetts vom Kolostrum, Gerinnen des Caseins des fettfreien Kolostrums durch enzymatische Gerinnung und enzymatisches Abbauen der Lactose, die in der so erhaltenen Molke enthalten ist, oder (b) durch Zusatz von Lactaseenzym zum Kolostrum, Gerinnen des Caseins mit enzymatischer Gerinnung und Trennen der so erhaltenen Molke,
- Einbringen eines Ansatzes der Molke/des Serums in eine Chromatographiesäule, die mit einem Kationenaustauscherharz gefüllt ist,
- Eluieren der Proteinfraktion von der Säule mit Wasser unterhalb einer Temperatur von 10 °C, und
- Sterilisieren, Konzentrieren und Gefriertrocknen der gewonnenen Proteinfraktion bis zu einem Trockenmassegehalt von etwa 10% bis etwa 30%.

10. Verfahren nach Anspruch 9, wobei das Harz ein starkes Kationenaustauscherharz in Na⁺-Form ist.

11. Verfahren nach Anspruch 9, wobei das Harz mit Molke equilibriert wird, bevor der Molkesatz in die Säule eingebracht wird.

12. Verfahren nach Anspruch 9, welches das Ausführen des Konzentrationsschritts mit umgekehrter Osmose umfasst, wobei die Konzentration 1:2 bis 1:3 bezüglich des Flüssigkeitsvolumens beträgt.

13. Veuahren nach Anspruch 9, welches das Lagern des gefriergetrockneten Pulvers bei -70°C umfasst.

## Revendications

1. Composition pharmaceutique ayant *in vivo* une activité bactéricide contre des bactéries du genre *Helicobacter,* ladite composition comprenant, sous la forme d'un concentré lyophilisé, des protéines de complément enrichies isolées à partir du lait, du colostrum ou du sang, les protéines de complément conservant leur activité biologique pendant l'isolement et l'enrichissement et étant capables de conserver ladite activité pendant le stockage.

2. Composition pharmaceutique selon la revendication 1, dans laquelle les protéines de complément sont enrichies à partir de colostrum de bovin.

3. Composition.pharmaceutique selon la revendication 1, dans laquelle les protéines de complément sont enrichies à partir de sérum de vertébrés ou dans laquelle le sérum ou les protéines de complément enrichies à partir de celui-ci est/sont ajouté(s) à la composition selon la revendication 2.

4. Composition pharmaceutique selon la revendication 1, 2 ou 3, dans laquelle les bactéries du genre *Helicobacter* appartiennent aux espèces *Helicobacter pylori, Helicobacter felis* ou *Helicobacter heilmannii.*

5. Composition pharmaceutique selon l'une quelconque des revendications 1 à 4, comprenant, en outre, des antibiotiques.

6. Utilisation d'une composition pharmaceutique comprenant, sous la forme d'un concentré lyophilisé, des protéines de complément enrichies isolées à partir du lait, du colostrum ou du sang, les protéines de complément conservant leur activité biologique pendant l'isolement et l'enrichissement et étant capables de conserver ladite activité pendant le stockage, pour la fabrication d'un médicament pour le traitement oral d'infections par un *Helicobacter* dans la muqueuse gastrique chez l'Homme ou un autre mammifère.

7. Utilisation selon la revendication 6, dans laquelle les bactéries du genre *Helicobacter* appartiennent aux espèces *Helicobacter pylori, Helicobacter felis* ou *Helicobacter heilmannii.*

8. Utilisation d'une composition pharmaceutique comprenant, sous la forme d'un concentré lyophilisé, des protéines de complément enrichies isolées à partir du lait, du colostrum ou du sang, les protéines de complément conservant leur activité biologique pendant l'isolement et l'enrichissement et étant capables de conserver ladite activité pendant le stockage, et comprenant, en outre, des antibiotiques, pour la fabrication d'un médicament pour le traitement oral d'infections par un *Helicobacter* dans la muqueuse gastrique chez l'Homme ou un autre mammifère.

9. Procédé d'enrichissement de protéines de complément à partir du lait ou du lactosérum colostral ou du sérum sanguin sous une forme biologiquement active, comprenant :
- le traitement préalable du lait, du colostrum ou du sang pour recueillir le lactosérum/sérum, moyennant quoi on obtient du lactosérum colostral en (a) séparant les matières grasses du colostrum, en faisant coaguler la caséine du colostrum débarrassé des matières grasses par coagulation enzymatique et en dégradant enzymatiquement le lactose inclus dans le lactosérum ainsi obtenu, ou (b) en ajoutant une enzyme lactase au colostrum, en faisant coaguler la caséine par coagulation enzymatique et en séparant le lactosérum ainsi obtenu,
- l'amenée d'un lot du lactosérum/sérum dans une colonne de chromatographie remplie d'une résine échangeuse de cations,
- l'élution de la fraction protéique à partir de la colonne avec de l'eau au-dessous de la température de 10°C, et
- la stérilisation, la concentration et la lyophilisation de la fraction protéique recueillie jusqu'à une teneur en matière sèche d'environ 10 à environ 30 %.

10. Procédé selon la revendication 9, dans lequel la résine est une résine fortement échangeuse de cations sous la forme de Na⁺.

11. Procédé selon la revendication 9, dans lequel on équilibre la résine avec du lactosérum avant d'amener le lot de lactosérum dans la colonne.

12. Procédé selon la revendication 9, comprenant la mise en oeuvre de l'étape de concentration par osmose inverse de 1:2 à 1:3 en ce qui concerne le volume de liquide.

13. Procédé selon la revendication 9, comprenant le stockage de la poudre lyophilisée à -70°C.
